# EUROPÄISCHE PATENTSCHRIFT

(11) **EP 1 940 306 B1**
(45) Veröffentlichungstag und Bekanntmachung des Hinweises auf die Patenterteilung: **07.09.2011**
(21) Anmeldenummer: 06804831.3
(22) Anmeldetag: 25.10.2006
(51) Int. Cl.: A61B 17/86

(54) **GEWINDEFORMENDE SCHRAUBE**
THREAD-FORMING SCREW
TARAUD POUR FORMER DES FILETS

(30) Priorität: 28.10.2005 CH 17312005
(43) Veröffentlichungstag der Anmeldung: 09.07.2008
(73) Patentinhaber: Medartis AG, 4057 Basel (CH)
(72) Erfinder: HASENBÖHLER, Alain, CH-4102 Binningen (CH); SCHEUBLE, Peter, 79418 Schliengen (DE); THIEL, Dirk, 79219 Staufen (DE)
(74) Vertreter: Hepp Wenger Ryffel AG
(86) Internationale Anmeldenummer: PCT/CH2006/000597
(87) Internationale Veröffentlichungsnummer: WO 2007/048267

(56) Entgegenhaltungen:
- WO-A-2004/086991
- FR-A- 2 808 182
- US-A- 6 030 162
- US-A1- 2003 120 279
- US-A1- 2004 230 195
- US-A1- 2005 101 961
- US-B1- 6 306 140

## Beschreibung

Die Erfindung betrifft eine Gewinde formende Schraube gemäss dem Oberbegriff des unabhängigen Patentanspruchs.

Gewinde formende Schrauben sind weithin bekannt in Form von selbstschneidenden oder selbstbohrenden Schrauben. Sie haben den Vorteil, dass kein Gewinde vorgeschnitten werden muss. Eine derartige Schraube ist beispielsweise aus der DE 20 2004 011 145 U1 bekannt und wird dort als Steinschraube, beispielsweise als Backsteinschraube eingesetzt. Die dort beschriebene Schraube hat einen Vorformbereich mit einem Vorgewinde, an welchen sich ein erster Gewindeabschnitt, danach ein gewindeloser Abschnitt, und dann mindestens ein weiterer Gewindeabschnitt anschliesst, der unterhalb des Schraubenkopfs angeordnet ist.

Selbstschneidende und selbstbohrende Schrauben werden auch auf anderen Gebieten eingesetzt, beispielsweise in der Medizintechnik, und hier insbesondere im Bereich der Osteosynthese (beispielsweise im Schädelbereich, im Mund-Kiefer-Gesicht-Bereich, im Handbereich oder in der Unfallchirurgie). Die dabei verwendeten Schrauben sind Schrauben, deren Schaft über die gesamte Schaftlänge (mit Ausnahme des spitzen oder stumpfen Schraubenendes) zylindrisch oder konisch ausgebildet sind und die mit einem durchgehenden Gewinde konstanter Steigung versehen sind. Mit Hilfe dieser Schrauben werden Hilfsmittel wie z.B. Knochenplatte oder Platten von Distraktoren befestigt, die in der Osteosynthese nicht mehr wegzudenken sind.

Bei Anwendungen im Schädelbereich bzw. speziell im Mund-Kiefer-Gesicht-Bereich sind die Durchmesser solcher Schrauben naturgemäss möglichst klein. Da die Schrauben aber fest im Knochen verankert werden müssen, weisen sie oft eine nicht unerhebliche Schaftlänge auf, erst recht, wenn man eine transmukosale oder transgingivale Befestigung in Betracht zieht, und also eine chirurgische Darstellung des Knochens (durch Aufschneiden und Wegklappen von Mukosa bzw. Gingiva) vermeiden möchte.

Allerdings können beim Eindrehen solcher Schrauben einige Schwierigkeiten auftreten. Beispielsweise wächst das Drehmoment mit zunehmender Eindrehtiefe ganz erheblich an, was insbesondere bei längeren Schrauben das Aufbringen von ganz erheblichen Kräften durch den Chirurgen erfordert, und das bei einem typischerweise sehr beengten Operationsfeld. Dies gilt sowohl für selbstschneidende Schrauben, für die in einem separaten Schritt zuvor eine Kernbohrung im Knochen erstellt worden ist, aber erst recht für selbstbohrende Schrauben, die ohne Vorbohren in den Knochen geschraubt werden, was für den Chirurgen einen Arbeitsschritt (nämlich das Vorbohren) einspart und sogar zu einer besseren Verankerung der Schraube im Knochen führen kann.

Ausserdem kann beim Aufbringen der mit zunehmender Eindrehtiefe immer grösser werdenden erforderlichen Drehmomente der doch verhältnismässig dünne Schraubenschaft reissen (Torsionsbruch), sodass der bereits im Knochen steckende Rest des Schraubenschafts in einem solchen Fall aufwändig entfernt werden muss. Dem kann man zwar bei selbstschneidenden Schrauben insofern etwas vorbeugen, indem man den Durchmesser der Kernbohrung erhöht. Dazu ist aber entweder ein weiterer Arbeitsschritt erforderlich (Aufweiten der ursprünglichen Kernbohrung mit einem Bohrer mit grösserem Durchmesser) oder es wird gleich mit einem Bohrer mit grösserem Durchmesser gebohrt, wobei dann die Gefahr besteht, dass der Bohrer verwechselt werden kann und nicht mit dem richtigen Bohrer gebohrt wird. Ausserdem ist die Verankerung der Schraube im Knochen bei seinem grösseren Durchmesser der Kernbohrung natürlich geringfügig schlechter.

Aus der US 6 030 162 A ist eine Kompressionsschraube bekannt, die entlang ihrer Länge Gewindegänge mit unterschiedlicher Steigung und unterschiedlichem Aussendurchmessen aufweist. Aufgrund dieser unterschiedlichen Steigungen wird eine axiale Kompression zwischen einem vorderen und einem hinteren Abschnitt der Schraube erzeugt.

Sowohl der zusätzliche Arbeitsschritt des nochmaligen Vorbohrens als auch die Verwechselungsgefahr bei der Auswahl des Bohrers wie auch das Aufbringen von teilweise sehr grossen Kräften zur Erzeugung des erforderlichen Drehmoments sind für den Chirurgen nachteilig. Hier setzt die vorliegende Erfindung an, deren Aufgabe es ist, eine Schraube vorzuschlagen, die diesbezüglich Abhilfe leisten kann.

Diese Aufgabe wird erfindungsgemäss durch eine Schraube gelöst, wie sie durch die Merkmale des unabhängigen Patentanspruchs charakterisiert ist. Vorteilhafte Weiterbildungen der erfindungsgemässen Schraube sind Gegenstand der abhängigen Patentansprüche.

Speziell wird eine Gewinde formende Schraube, insbesondere eine Knochenschraube vorgeschlagen, mit einem Schraubenende, einen Schaft und einem gewindefreien Kopf. Der Schaft ist zumindest teilweise mit einem Gewinde versehen und weist einen Vorformbereich, einen Zwischenbereich, der dem Vorformbereich nachfolgend angeordnet ist, sowie einen Verankerungsbereich auf. Der Verankerungsbereich ist anschliessend an den Zwischenbereich und unterhalb des Kopfs angeordnet. Der Vorformbereich ist unmittelbar an das Schraubenende anschliessend angeordnet. Beim Einschrauben des Vorformbereichs nimmt das zum Einschrauben erforderliche Drehmoment zu, bis der Vorformbereich der Schraube vollständig eingeschraubt ist. Vorformbereich, Zwischenbereich und Verankerungsbereich sind derart ausgebildet, dass nach vollständigem Einschrauben des Vorformbereichs entweder keine Zunahme des zum Einschrauben erforderlichen Drehmoments oder nur eine im Verhältnis zur Zunahme beim Einschrauben des Vorformbereichs geringe Zunahme des erforderlichen Drehmoments auftritt, bis die Schraube bis zu ihrem Verankerungsbereich eingeschraubt ist. Der Ausdruck "keine Zunahme des Drehmoments" soll hierbei auch solche Fälle umfassen, bei denen das erforderliche Drehmoment beim weiteren Einschrauben der Schraube nicht nur gleich bleiben kann, sondern sogar abnehmen kann. Dies kann beispielsweise der Fall sein, wenn der Vorformbereich durch die Kortikalis hindurch geschraubt worden ist und in die Spongiosa eindringt. Für das weitere Einschrauben des Vorformbereich in die Spongiosa muss dann ein geringeres Drehmoment aufgebracht werden als beim Einschrauben in die Kortikalis. Der Zwischenbereich der Schraube lässt sich ebenfalls mit einem geringeren Drehmoment in die Kortikalis einschrauben als der Vorformbereich, sodass die Summe der erforderlichen Drehmomente zum Einschrauben von Vorformbereich und Zwischenbereich in einem solchen Fall geringer sein kann als das Drehmoment, welches zum Einschrauben des Vorformbereichs in die Kortikalis erforderlich ist.

Hierdurch unterscheidet sich die erfindungsgemässe Schraube wesentlich von herkömmlichen Schrauben: Je nachdem, wie die Schraube ausgeführt ist, bleibt nach dem Einschrauben des Vorformbereichs (der sich nur über einen Teil der Länge des Schraubenschafts erstreckt) das Eindrehmoment entweder im Wesentlichen konstant, es findet also keine wesentliche Zunahme des erforderlichen Drehmoments mehr statt. In einigen Fällen (siehe oben) kann das erforderliche Drehmoment sogar abnehmen. Oder aber das erforderliche Drehmoment kann auch zunehmen (ausgeprägte Kortikalis), jedoch ist die Zunahme des erforderlichen Drehmoments in jedem Fall erheblich geringer als die Zunahme des Drehmoments beim Eindrehen von konventionellen Schrauben. Die Schraubenlänge hat also entweder keinen oder nur noch einen wesentlich reduzierten Einfluss auf das erforderliche Eindrehmoment, weshalb auch lange selbstbohrende Schrauben verwendet werden können, ohne dass die Gefahr des Reissens (Torsionsbruch) besteht. Ein Vorbohren für lange selbstbohrende Schrauben zur Verringerung des Risikos eines Torsionsbruchs ist somit nicht mehr nötig. Auch kann die erfindungsgemässe Schraube geeignet sein, um aus resorbierbaren Materialien hergestellt zu werden, mit denen sich eben nicht beliebig grosse Drehmomente übertragen lassen.

Beim Einschrauben des Verankerungsbereichs kann das erforderliche Drehmoment wieder stärker zunehmen. Das ist aber im Hinblick auf die Gefahr eines Torsionsbruchs der Schraube nicht so kritisch, weil der Kerndurchmesser im Verankerungsbereich der Schraube regelmässig genügend gross ist, um das erforderliche Drehmoment zuverlässig übertragen zu können.

Bei einem ersten Ausführungsbeispiel der erfindungsgemässen Schraube ist der Aussendurchmesser der Schraube im Vorformbereich zumindest in einem an den Zwischenbereichs angrenzenden Abschnitt grösser als im Zwischenbereich selbst. Ausserdem ist zumindest in einem Teil des Verankerungsbereichs der Aussendurchmesser gleich gross oder im Wesentlichen gleich gross wie der Aussendurchmesser des an den Zwischenbereich angrenzenden Abschnitts des Vorformbereichs.

Weil der Aussendurchmesser des Vorformbereichs dort, wo der Vorformbereich an den Zwischenbereich angrenzt, grösser ist als der Aussendurchmesser des Zwischenbereichs, läuft ₋ je nachdem, wie der Zwischenbereich ausgebildet ist - der Zwischenbereich frei oder die Reibung beim Eindrehen ist erheblich reduziert. Beispielsweise ist es denkbar, den Zwischenbereich ganz ohne Gewinde auszubilden und mit einem Kerndurchmesser zu versehen, der etwas kleiner ist als der Kerndurchmesser des Vorformbereichs dort, wo dieser an den Zwischenbereich angrenzt. Dann kann der Zwischenbereich beim Eindrehen frei laufen und es erfolgt keine Zunahme des Eindrehmoments. Allerdings hat die Schraube dann im Zwischenbereich auch keinerlei Halt im Knochen. Wird im Zwischenbereich ebenfalls ein Gewinde vorgesehen, jedoch mit einem geringeren Aussendurchmesser als im Vorformbereich (dort, wo dieser an den Zwischenbereich angrenzt), kann beim Eindrehen der Schraube ein zusätzliches Eindrehmoment erforderlich werden. Allerdings ist die Zunahme des Eindrehmoments wesentlich geringer als bei konventionellen Schrauben. Dafür hat die Schraube im Knochen auch einen besseren Halt und bietet beispielsweise einen Widerstand gegen axiales Ausreissen. Ausserdem kann der Knochen während des Heilungsprozesses zwischen den Gewindespitzen in den Gewindegrund einwachsen, was die Verankerung der Schraube im Knochen weiter verbessert.

Bei einem zweiten Ausführungsbeispiel der erfindungsgemässen Schraube weist der Schraubenschaft im Vorformbereich und im Zwischenbereich einen konstanten Aussendurchmesser auf. Im Vorformbereich ist er mit einem Gewinde mit verschiedenen Steigungen versehen, nämlich einer ersten Steigung und einer zweiten Steigung, wobei die zweite Steigung grösser ist als die erste Steigung. Im verankerungsbereich - und vorzugsweise auch im Zwischenbereich- ist der Schraubenschaft mit einem Gewinde mit einer dritten steigung versehen, die grösser ist als die erste Steigung und kleiner als die zweite Steigung des Gewindes im Vorformbereich. Beim Einschrauben wird das Gewinde, welches durch die erste Gewindesteigung geschnitten wird, anschliessend durch die zweite (grossere) Gewindesteigung ein wenig "aufgeweitet" (in axialer Richtung verbreitert). In diesen verbreiterten Gewindegang hinein kommen dann die nachfolgenden Gewindegänge mit der dritten

Steigung zu liegen, sie haben in axialer Richtung also ein geringes Spiel, weshalb die zusätzliche Reibung beim Einschrauben allenfalls gering ist.

Bei einer Weiterbildung der erfindungsgemässen Schraube ist das Schraubenende stumpf ausgebildet. Es handelt sich bei dieser Schraube also um eine selbstschneidende (nicht selbstbohrende) Schraube, es wird also eine Vorbohrung im Knochen erstellt, bevor die selbstschneidende Schraube dann in diese Vorbohrung eingeschraubt wird und das Gewinde in den Knochen schneidet. Der Aussendurchmesser der Schraube ist im Vorformbereich konstant (zylindrischer Vorformbereich). Im Zwischenbereich ist er in einem unmittelbar an den Vorformbereich angrenzenden Abschnitt kleiner als der Aussendurchmesser des Vorformbereichs. Ausgehend von diesem an den Vorformbereich angrenzenden Abschnitt des Zwischenbereichs nimmt der Aussendurchmesser über den Zwischenbereich und den Verankerungsbereich hinweg konisch zu, bis er im Verankerungsbereich gleich gross oder im Wesentlichen gleich gross ist wie der Aussendurchmesser des Vorformbereichs. Das heisst, dass nach dem Einschrauben des Vorformbereichs beim weiteren Einschrauben allenfalls nur ein wesentlich geringeres zusätzliches Drehmoment aufgebracht werden muss als bei konventionellen Schrauben. Am Ende des Einschraubvorgangs kommt der Verankerungsbereich der Schraube in der Kortikalis zu liegen, und da der Verankerungsbereich zumindest in einem Teil einen gleich grossen oder im Wesentlichen gleich grossen Aussendurchmesser aufweist wie der Vorformbereich, ist die Schraube dann gut in der Kortikalis verankert.

Bei einer anderen Weiterbildung der erfindungsgemässen Schraube ist das Schraubenende als Spitze ausgebildet. Es handelt sich bei diesem Ausführungsbeispiel um eine selbstbohrende (und natürlich auch selbstschneidende) Schraube. Eine Vorbohrung im Knochen braucht hier - im Unterschied zur lediglich selbstschneidenden Schraube - nicht erstellt zu werden. Der Aussendurchmesser des Vorformbereichs nimmt ausgehend von der Spitze bis zu demjenigen Abschnitt, der unmittelbar an den Zwischenbereich angrenzt, zu. Im Zwischenbereich ist der Aussendurchmesser in einem unmittelbar an den Vorformbereich angrenzenden Abschnitt kleiner als der Aussendurchmesser des angrenzenden Abschnitts des Vorformbereichs. Ausgehend von diesem an den Vorformbereich angrenzenden Abschnitt des Zwischenbereichs nimmt der Aussendurchmesser über den Zwischenbereich und den Verankerungsbereich hinweg konisch zu, bis er zumindest in einem Teil des Verankerungsbereichs gleich gross ist oder im Wesentlichen gleich gross wie der Aussendurchmesser des Vorformbereichs in dem an den Zwischenbereich angrenzenden Abschnitt.

Durch die Spitze und den zunächst kleinen Durchmesser des Vorformbereichs lässt sich die selbstbohrende Schraube gut ansetzen und zu Beginn des Einschraubens auch ganz leicht eindrehen, um ein Abgleiten der Schraube zu verhindern. Mit der Zunahme des Aussendurchmessers des Vorformbereichs nimmt auch das aufzubringende Drehmoment zu, bis der Vorformbereich vollständig eingeschraubt ist. Nach vollständigem Einschrauben des Vorformbereichs nimmt beim weiteren Einschrauben das aufzubringende Drehmoment allenfalls wesentlich geringer zu als bei konventionellen Schrauben, weil im Zwischenbereich kein vollständiger Kontakt mit dem Knochen besteht und daher auch die zusätzliche Reibung geringer ist. Am Ende des Einschraubvorgangs kommt der Verankerungsbereich der Schraube in der Kortikalis zu liegen, und da der Verankerungsbereich zumindest in einem Teil einen gleich grossen oder im Wesentlichen gleich grossen Aussendurchmesser aufweist wie der Vorformbereich, ist die Schraube dann gut in der Kortikalis verankert.

Bei einer Weiterbildung der erfindungsgemässen Schraube weist der Schraubenschaft im Vorformbereich einen polygonartigen Querschnitt auf, der Punkte bzw. Bereiche auf weist, die einen maximalen radialen Abstand von der Schraubenachse haben und Bereiche, die einen geringeren radialen Abstand von der Schraubenachse haben als diejenigen Punkte bzw. Bereiche mit maximalem radialen Abstand von der Schraubenachse. Beim Vorformen des Gewindes wird also immer an den Punkten bzw. Bereichen, die einen maximalen Abstand von der Schraubenachse haben, die Reibung beim Gewindeformen maximal, während sie in den dazwischen liegenden Bereichen geringer wird. Dadurch wird das zum Einschrauben des Vorformbereichs erforderliche Drehmoment noch einmal verringert. Beispielsweise kann der Schraubenschaft im Vorformbereich als sogenannter Gleichdick ausgebildet sein, der eine dreieckähnliche äussere Gestalt (wird später noch genauer erläutert) aufweist.

Wie bereits weiter oben erwähnt, ist bei einem Ausführungsbeispiel der erfindungsgemässen Schraube der Aussendurchmesser im Zwischenbereich kleiner als in demjenigen Abschnitt des Vorformbereichs, der an den Zwischenbereich angrenzt. Dazu kann beispielsweise der Kerndurchmesser des Schraubenschafts im Zwischenbereich kleiner sein als in dem an den Zwischenbereich angrenzenden Abschnitt des Vorformbereichs. Hier ist allerdings darauf zu achten, dass der Kerndurchmesser nicht beliebig klein gewählt werden kann, nur um ein möglichst einfaches Einschrauben des Zwischenbereichs zu erwirken. Stattdessen ist darauf zu achten, dass der reduzierte Kerndurchmesser im Zwischenbereich - vor allem an des Übergang zum Vorformbereich - stets so gewählt ist, dass das zum Einschrauben erforderliche Drehmoment auch von der schwächsten Stelle der Schraube sicher übertragen wird, um ein Reissen (Torsionsbruch) der Schraube sicher zu verhindern. In demjenigen Teil des Verankerungsbereichs, der den gleich grossen oder im Wesentlichen gleich grossen Aussendurchmesser aufweist wie der an den zwischenbereich angrenzende Abschnitt des Vorformbereichs, ist der Kerndurchmesser des Schraubenschafts gleich gross oder im Wesentlichen gleich gross wie der Kerndurchmesser des an den Zwischenbereich angrenzenden Abschnitts des Vorformbereichs, sodass die Schraube dann gut in der Kortikalis verankert ist.

Die erfindungsgemässe Schraube ist vorzugsweise aus einem biokompatiblen Material hergestellt, beispielsweise aus Titan oder einer Titanlegierung, oder aus einem bioresorbierbaren Material (späteres Ausdrehen der Schraube nicht mehr erforderlich) was vor allen Dingen dann wichtig ist, wenn sie als Knochenschraube eingesetzt wird und somit aus einem bioverträglichen Material hergestellt sein muss. Allerdings ist dies nicht der einzig denkbare Anwendungsbereich, sodass das Material nicht zwingend körperverträglich sein murs.

Weitere vorteilhafte Aspekte der erfindungsgemässen Schraube ergeben sich aus der nachfolgenden Beschreibung von Ausführungsbeispielen der Erfindung mit Eilfe der Zeichnung.

Es zeigen:
- Fig. 1: eine Ansicht eines ersten Ausführungsbeispiels der erfindungsgemässen Schraube,
- Fig. 2: die Ansicht aus Fig. 1 mit einigen Eilfslinien zur besseren raumlichen Darstellung der Schraube,
- Fig. 3: eine schematische Darstellung der Schraube aus Fig. 1 mit übertriebener Darstellung des Kerns. des Schraubenschafts,
- Fig. 4: eine schematische Darstellung des Kerns des Schraubenschafts der Schraube aus Fig. 1,
- Fig. 5: eine schematische Darstellung verschiedener Positionen der Schraube aus Fig. 1 beim Einschrauben,
- Fig. 6: eine Ansicht eines zweiten Ausführungsbei- spiels der erfindungsgemässen Schraube,
- Fig. 7: die Ansicht aus Fig. 6 mit einigen Hilfslinien zur besseren räumlichen Darstellung der Schraube,
- Fig. 8: eine schematische Darstellung der Schraube aus Fig. 6 mit übertriebener Darstellung des Kerns des Schraubenschafts,
- Fig. 9: eine schematische Darstellung des Kerns des Schraubenschafts der Schraube aus Fig. 6,
- Fig. 10: eine Ansicht der Schraube aus Fig. 6 von unten,
- Fig. 11: eine schematische Darstellung verschiedener Positionen der Schraube aus Fig. 6 beim Einschrauben,
- Fig. 12: eine Ansicht eines dritten Ausführungsbei- spiels der erfindungsgemässen Schraube,
- Fig. 13: eine schematische Darstellung des Kerns des Schraubenschafts der Schraube aus Fig. 12,
- Fig. 14: eine schematische Ansicht eines Querschnitts durch einen sogenannten "Gleichdick" und eines zugehörigen Abschnitts des Vorformbereichs in einer Ansicht,
- Fig. 15: eine Ansicht der Schraube aus Fig. 12 von unten,
- Fig. 16: eine schematische Darstellung verschiedener Positionen der Schraube aus Fig. 12 beim Einschrauben,
- Fig. 17: eine Ansicht eines vierten Ausführungsbei- spiels der erfindungsgemässen Schraube, und
- Fig. 18: eine schematische Darstellung der Schraube aus Fig. 17 in eingeschraubtem Zustand.

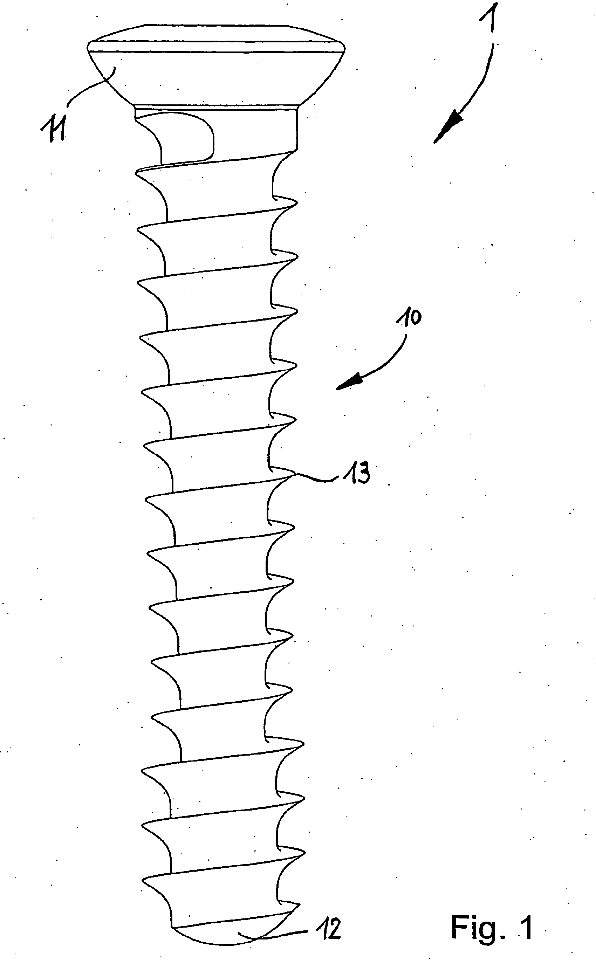

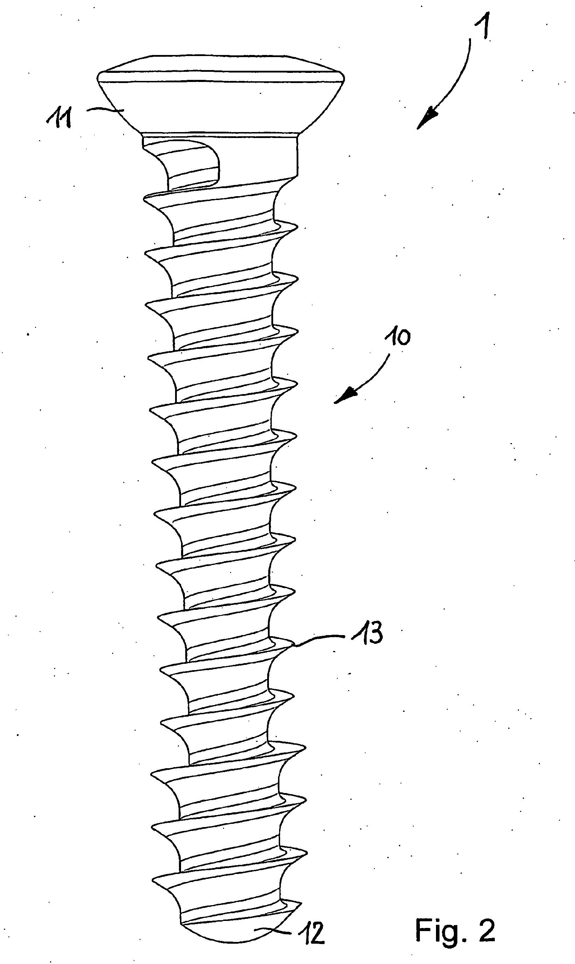

Ein erstes Ausführungsbeispiel der erfindungsgemässen Schraube wird im folgenden anhand von Fig. 1 bis Fig. 5 erläutert. Fig. 1 zeigt eine Ansicht des ersten Ausführungsbeispiels der erfindungsgemässen Schraube 1, hier einer Knochenschraube. Die Schraube 1 umfasst einen Schaft 10 sowie einen gewindefreien sphärischen Kopf 11, welcher beispielsweise erlaubt, in einem angesenkten Plattenloch einer Knochenplatte (nicht dargestellt) aufgenommen zu werden. Das Schraubenende 12 ist stumpf ausgebildet, es handelt sich somit bei der in Fig. l gezeigten Schraube 1 um eine selbstschneidende (nicht selbstbohrende) Schraube. Fig. 2 zeigt die gleiche Schraube wie Fig. 1, jedoch ergänzt um einige Hilfslinien, wodurch man eine besseren räumlichen Eindruck von der Schraube 1 erhält.

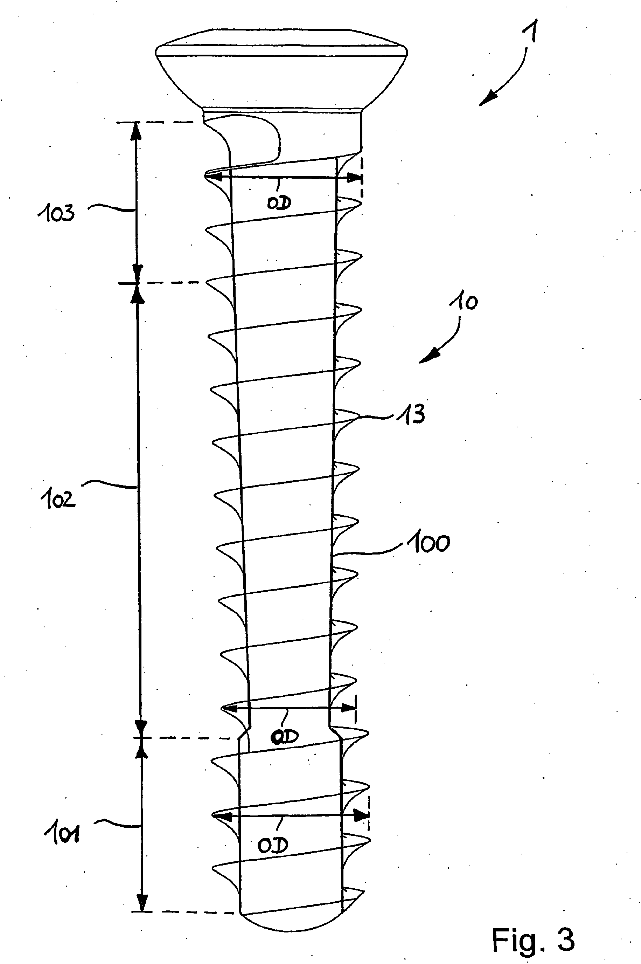

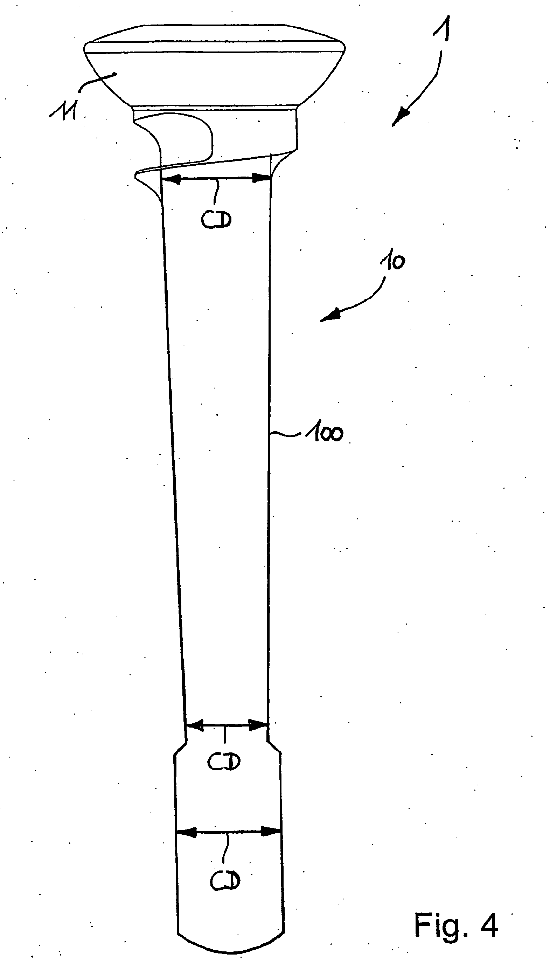

Die Einhüllende über das Gewinde 13 der Schraube, welches hier als durchgängiges Gewinde mit konstanter Steigung ausgebildet ist, kann man anhand von Fig. 3 erahnen, obwohl in Fig. 3 - in übertriebener Darstellung - nur der Kern 100 des Schafts 10 dargestellt ist. Da das Gewinde 13 jedoch eine konstante radiale Gewindehöhe aufweist, verläuft die (nicht dargestellte) Einhüllende - die den Aussendurchmesser der Schraube repräsentiert - über das gesamte Gewinde 13 parallel zum Kern 100 des Schafts 10. Der Kern 100 alleine ist noch einmal schematisch in Fig. 4 dargestellt.

Der Schaft 10 weist drei Bereiche auf: Einen Vorformbereich 101, einen Zwischenbereich 102, sowie einen Verankerungsbereich 103, welche Bereiche man am besten in der Darstellung in Fig. 3 erkennen kann. Der Vorformbereich 101 ist bei diesem Ausführungsbeispiel zylindrisch ausgebildet, sodass der Aussendurchmesser OD der Schraube 1 (die radiale Gewindehöhe ist ja konstant) im gesamten Vorformbereich 101 konstant ist. In dem unmittelbar angrenzenden Abschnitt des Zwischenbereichs 102 ist der Aussendurchmesser OD kleiner als im Vorformbereich 101. Ausgehend von diesem an den Vorformbereich 101 angrenzenden Abschnitt des Zwischenbereichs 102 nimmt der Aussendurchmesser OD über den Zwischenbereich 102 und den Verankerungsbereich 103 hinweg konisch zu, bis er am Beginn des Verankerungsbereich 103 beinahe gleich gross und am oberen Ende des Verankerungsbereichs 103 gleich gross ist wie im Vorformbereich 101.

Dies gilt analog für den Kerndurchmesser CD des Schafts 10 (siehe Fig. 4). Im Vorformbereich ist der Kerndurchmesser CD grösser als in dem unmittelbar daran angrenzenden Abschnitt des Zwischenbereichs, und von dort aus nimmt er über den Zwischenbereich und den Verankerungsbereich hinweg konisch zu.

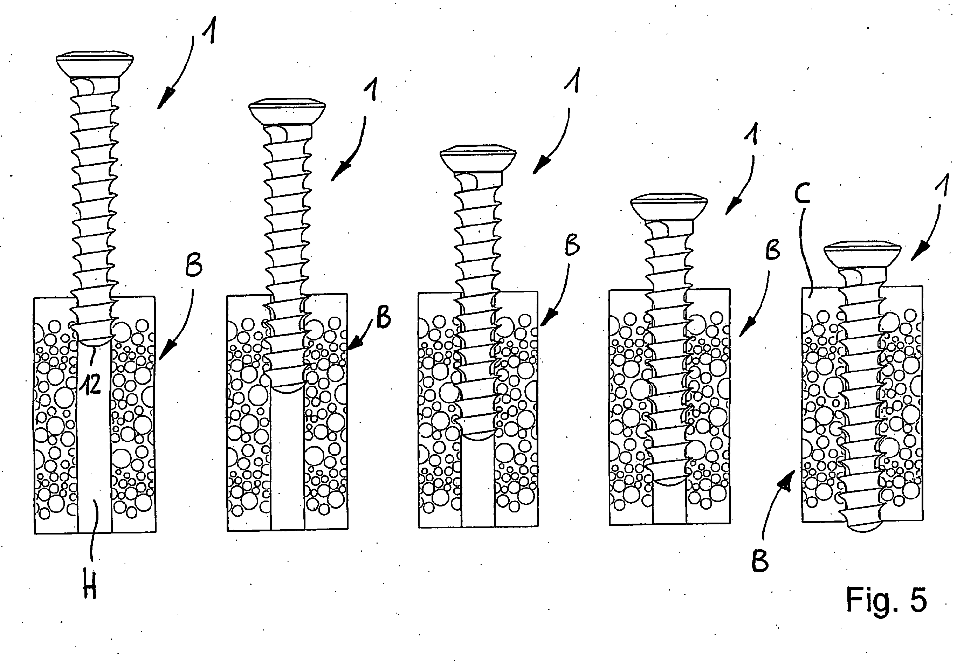

Zum Einschrauben einer solchen Schraube 1, die selbstschneidend (aber nicht selbstbohrend) ist, wird wie in Fig. 5 dargestellt vorgegangen. Zunächst wird eine (Vor) - Bohrung H erstellt, deren Bohrungsdurchmesser natürlich kleiner ist als der Aussendurchmesser der Schraube 1, aber grösser als der Kerndurchmesser der Schraube 1 in deren Vorformbereich 101. Die Schraube 1 wird dann mit dem stumpfen Schraubenende 12 in die Bohrung H eingesetzt und der Vorformbereich 101 wird sukzessive in den Knochen B eingeschraubt (linkes Bild in Fig. 5), zunächst in die Kortikalis. Beim Einschrauben wird ein Knochengewinde im Knochen. B geformt (in den Knochen "geschnitten"; selbstschneidend). Ist der Vorformbereich 101 vollständig in den Knochen B eingeschraubt, taucht dann der unmittelbar angrenzende Abschnitt des Zwischenbereichs 102 in den Knochen B ein. Da der Aussendurchmesser OD des Vorformbereichs 101 grösser ist als der Aussendurchmesser OD des unmittelbar angrenzenden Abschnitts des Zwischenbereichs 102, weist auch das von dem Vorformbereich 101 geschnittene Knochengewinde einen Durchmesser auf, der grösser ist als der Aussendurchmesser OD der Schraube im Zwischenbereich 102. Zwischen dem Gewinde des Zwischenbereichs 102 und dem von dem Vorformbereich 101 geschnittenen Knochengewinde besteht also ein radiales Spiel (zweites Bild von links in Fig. 5). Zwar können ggf. die Gewindeflanken in axialer Richtung leicht an dem von dem Vorformbereich 101 geschnittene Knochengewinde reiben, in radialer Richtung läuft das Gewinde im Zwischenbereich jedoch frei. Da der Aussendurchmesser der Schraube 1 des Zwischenbereichs konisch zunimmt, wird dieses radiale Spiel beim weiteren Einschrauben der Schraube 1 in den Knochen B wieder kleiner (drittes und viertes Bild von links in Fig. 5). Läuft die Schraube 3 im Zwischenbereich völlig frei (also auch ohne wesentliche axiale Reibung), ist zum weiteren Einschrauben der Schraube 1 in den Knochen B lediglich das Drehmoment zum Einschrauben des Vorformbereichs 101 erforderlich. Tritt noch eine geringe zusätzliche Reibung auf, wird das zum Einschrauben erforderliche Drehmoment zwar zunehmen, aber doch in erheblich geringerem Masse als dies bei konventionellen Schrauben der Fall ist.

Für den Fall, dass der Vorformbereich der Schraube 1 bereits in die Spongiosa eingetreten ist und das Gewinde des Zwischenbereichs reibungsfrei oder nur mit geringer Reibung läuft, kann das zum weiteren Einschrauben der Schraube 1 erforderliche Drehmoment sogar geringer werden. Denn das zum Einschrauben des Vorformbereichs 101 in die Spongiosa erforderliche Drehmoment ist geringer als das Drehmoment, welches erforderlich war, um den Vorformbereich in die Kortikalis einzuschrauben, und das Gewinde des Zwischenbereichs 102 läuft reibungsfrei oder nur mit geringer Reibung. Das erforderliche Drehmoment kann allerdings wieder stärker zunehmen, wenn die Schraube bis zum Verankerungsbereich 103 eingeschraubt ist und nun der Verankerungsbereich 103 der Schraube in Eingriff mit dem Knochen B gelangt, weil in diesem Bereich die Reibung des Gewindes mit dem Knochen B wieder zunimmt. Dabei kann der Verankerungsbereich 103 grundsätzlich sogar vom Aussendurchmesser her grösser sein als der Vorformbereich 101, um eine besonders gute Verankerung der Schraube 1 in der Kortikalis des Knochens B zu erreichen. Dies gilt analog auch für die anderen Ausführungsbeispiele der erfindungsgemässen Schraube.

Ist die Schraube 1 weitgehend in den Knochen B eingeschraubt (rechtes Bild in Fig. 5, Darstellung ohne Knochenplatte), ist der Verankerungsbereich 103 der Schraube 1 in der Kortikalis C (harte Knochenrinde) gut verankert, da der Aussendurchmesser OD der Schraube 1 im Verankerungsbereich 103 ja gleich gross oder im Wesentlichen gleich gross ist wie der Aussendurchmesser OD des Vorformbereichs 101 und damit gleich gross wie der Durchmesser des Knochengewindes. Damit ist das radiale Spiel dort null bzw. vernachlässigbar klein. Der Kopf der Schraube kann alternativ zu dem dargestellten sphärischen Kopf 11 auch so ausgebildet sein, wie dies beispielsweise in der WO 2004/086990 beschrieben ist, sodass man dann beispielsweise eine Knochenplatte in geringem Abstand vom Knochen fixieren kann (quasi als "fixateur intern"). Dies gilt analog auch für die nachfolgend noch erläuterten Ausführungsbeispiele der erfindungsgemässen Schraube.

Insgesamt lässt sich dieses Ausführungsbeispiel der erfindungsgemässen Schraube 1 also wesentlich leichter in den Knochen B einschrauben als konventionelle Schrauben, wodurch der Aufwand für den Chirurgen reduziert wird, aber vor allem auch das Risiko eines Torsionsbruchs verringert wird, vor allem bei langen und vergleichsweise "dünnen" Schrauben (also z.B. bei Schrauben für den Mund-Kiefer-Gesicht-Bereich mit einem Aussendurchmesser von 2.0 mm und einer Länge ab 10 mm). Bei der Bemessung des Durchmessers des Kerns 100 des Schafts 10 - insbesondere im an den Vorformbereich angrenzenden Abschnitt des Zwischenbereichs - ist also immer darauf zu achten, dass das zum Einschrauben erforderliche Drehmoment sicher übertragen werden kann, ohne dass die Schraube reissen kann (Torsionsbruch). Das heisst, dass der Kerndurchmesser CD in dem Abschnitt des Zwischenbereichs 102, der unmittelbar an den Vorformbereich 101 angrenzt, nicht einfach beliebig verringert werden kann, um die Reibung zu reduzieren und damit das zusätzlich erforderliche Drehmoment beim weiteren Einschrauben zu reduzieren, sondern vielmehr ist darauf zu achten, dass das erforderliche Drehmoment in jedem Fall über die gesamte Länge des Schafts 10 sicher übertragen werden kann.

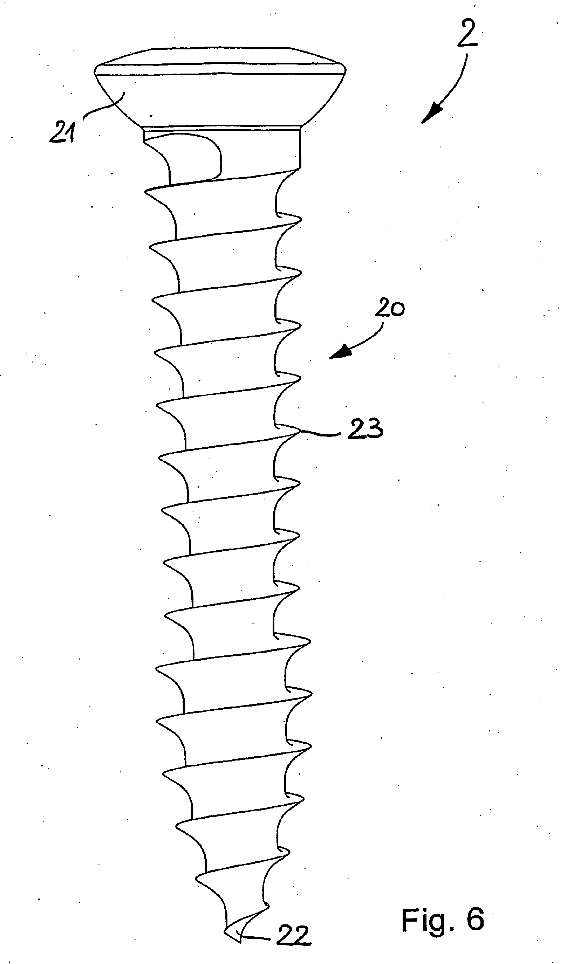

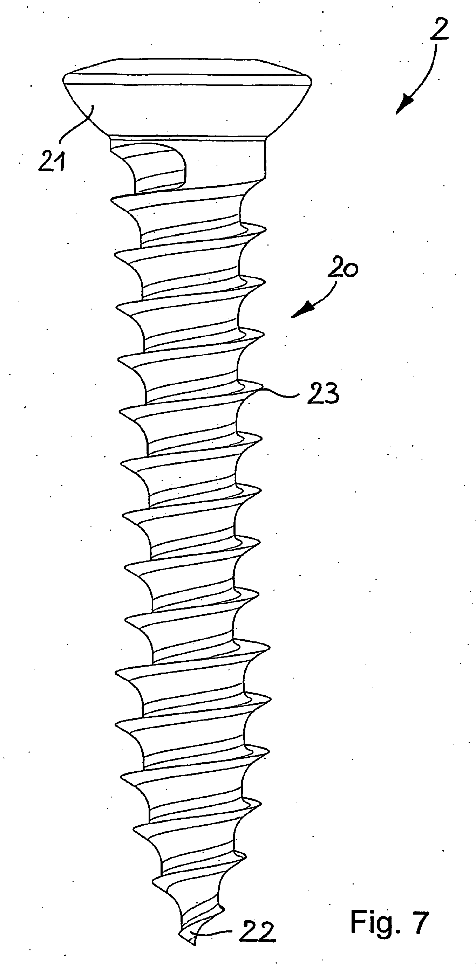

Ein zweites Ausführungsbeispiel der erfindungsgemässen Schraube wird im folgenden anhand von Fig. 6 bis Fig. 11 erläutert. Fig. 6 zeigt eine Ansicht des zweiten Ausführungsbeispiels der erfindungsgemässen Schraube 2, erneut einer Knochenschraube. Die Schraube 2 umfasst einen Schaft 20 sowie einen gewindefreien Senkkopf 21, welcher beispielsweise erlaubt, in einem angesenkten Plattenloch einer Knochenplatte (nicht dargestellt) aufgenommen zu werden. Das Schraubenende 22 ist als Spitze ausgebildet, es handelt sich somit bei der in Fig. 6 gezeigten Schraube 2 um eine selbstbohrende (und natürlich auch selbstschneidende) und selbstzentrierende Schraube. **Fig. 7** zeigt die gleiche Schraube, ergänzt um einige Hilfslinien, wodurch man eine besseren räumlichen Eindruck von der Schraube 2 erhält.

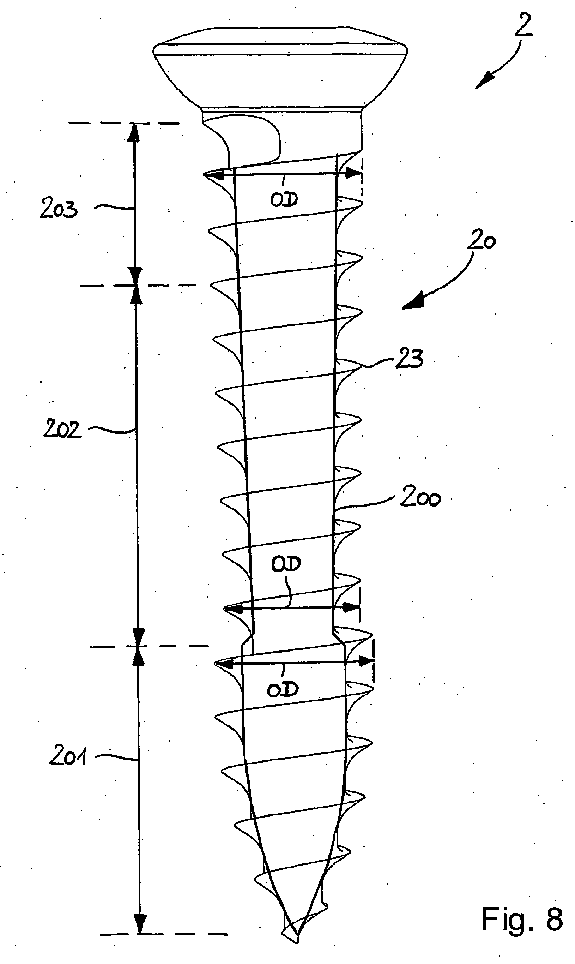

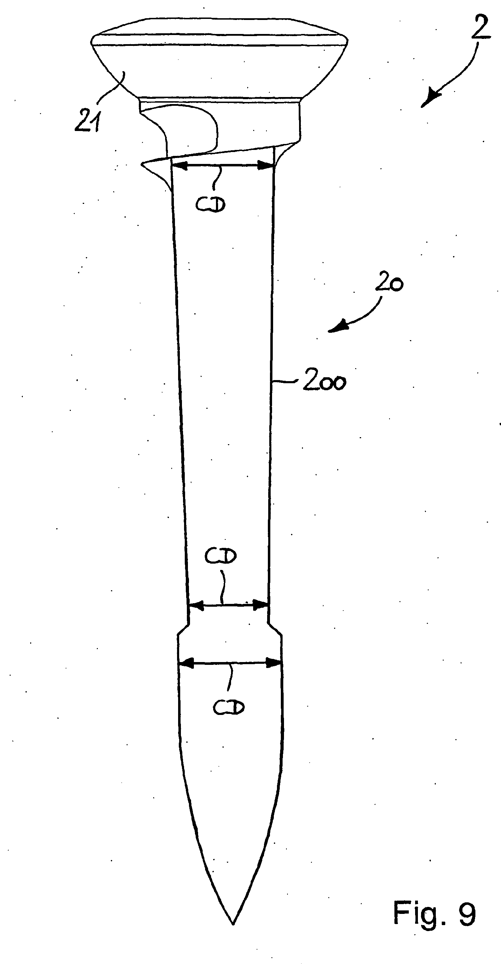

Die Einhüllende über das Gewinde 23 der Schraube, welches hier als durchgängiges Gewinde mit konstanter Steigung ausgebildet ist, kann man anhand von **Fig. 8** erahnen, obwohl in Fig. 8 in übertriebener Darstellung der Kern 200 des Schafts 20 dargestellt ist. Da das Gewinde 23 erneut eine konstante radiale Gewindehöhe aufweist, verläuft die (nicht dargestellte) Einhüllende - die den Aussendurchmesser der Schraube repräsentiert - über das gesamte Gewinde 23 parallel zum Kern 200 des Schafts 20. Der Kern 200 alleine ist noch einmal schematisch in Fig. 9 dargestellt.

Der Schaft 20 weist erneut drei Bereiche auf: Einen Vorformbereich 201, einen Zwischenbereich 202, sowie einen Verankerungsbereich 203, die man am besten in der Garstellung in Fig. 8 erkennen kann. Im Vorformbereich 201 nimmt der Aussendurchmesser OD der Schraube 2 ausgehend von der Spitze 22 bis hin zu demjenigen Abschnitt, der an den Zwischenbereich 202 angrenzt, zu. In dem unmittelbar an diesen Abschnitt des Vorformbereichs 201 angrenzenden Abschnitt des Zwischenbereichs 202 ist der Aussendurchmesser OD kleiner als im Vorformbereich 201. Ausgehend von diesem an den Vorformbereich 201 angrenzenden Abschnitt des Zwischenbereichs 202 nimmt der Aussendurchmesser OD über den Zwischenbereich 202 und den Verankerungsbereich 103 hinweg wieder konisch zu, bis er am Beginn des Verankerungsbereichs :203 beinahe gleich gross und am oberen Ende des Verankerungsbereichs 203 gleich gross ist wie im Vorformbereich 201.

Dies gilt analog für den Kerndurchmesser CD des Schafts 20 (siehe Fig. 9). Im unmittelbar an den Zwischenbereich angrenzenden Abschnitt des Vorformbereichs ist der Kerndurchmesser CD grösser als in dem unmittelbar daran angrenzenden Abschnitt des Zwischenbereichs, und von dort aus nimmt er über den Zwischenbereich und den Verankerungsbereich hinweg konisch zu. Unter "konischer" Zunahme des Durchmessers sollen in dieser Anmeldung - wie hier gezeigt - geradlinig konische Zunahmen des Durchmessers verstanden sein wie auch andere Verläufe der Zunahme des Durchmessers, wie beispielsweise parabolische, hyperbolische, einer Wurzelfunktion folgende, oder sonstige Zunahmen des Durchmessers.

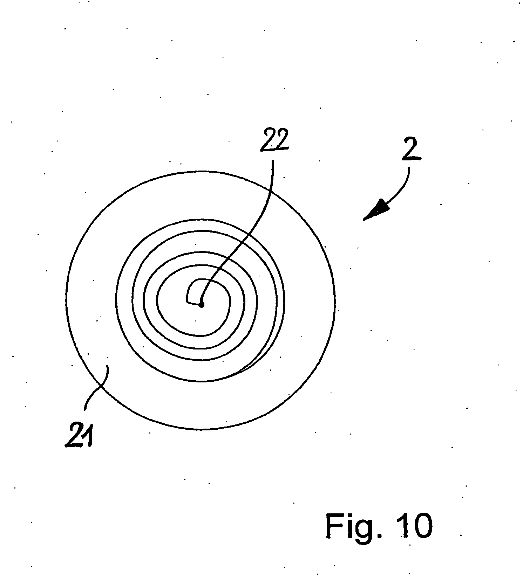

**Fig. 10** zeigt die Schraube 2 in einer Ansicht von unten, in welcher man gut die Zunahme des Aussendurchmessers von der Spitze 22 weg erkennt, bis er seinen maximalen Aussendurchmesser erreicht in dem Abschnitt des Vorformbereichs 201 (siehe Fig. 8), der an den Zwischenbereich 202 angrenzt.

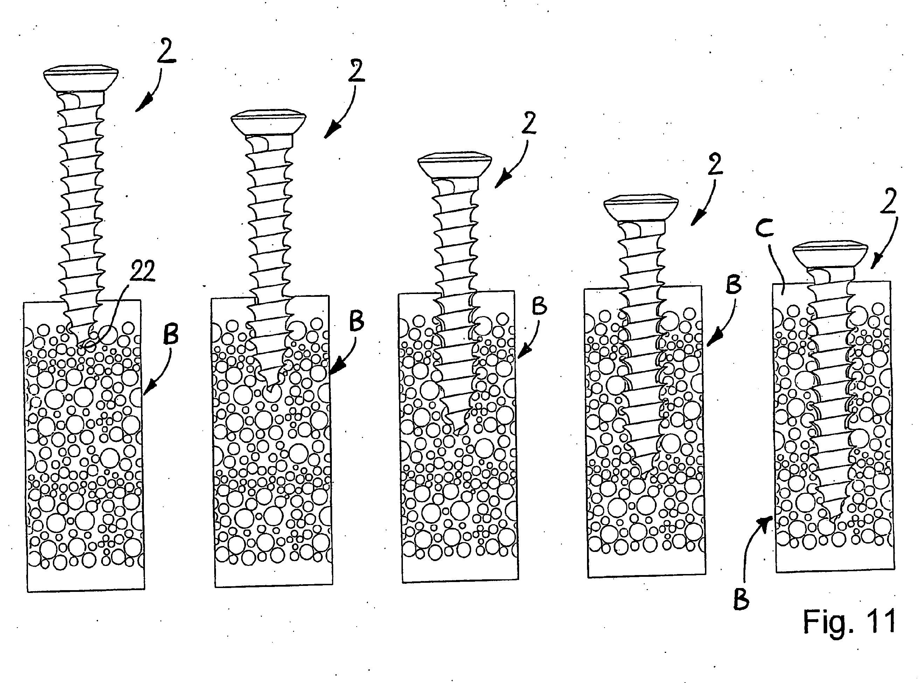

Zum Einschrauben einer solchen Schraube 2, die selbstbohrend ist, wird wie in Fig. 11 dargestellt vorgegangen. Zunächst wird die Schraube 2 mit der Spitze 22 auf den Knochen B aufgesetzt und eingeschraubt. Die Spitze 22 verhindert ein Abgleiten der Schraube, und der geringe Aussendurchmesser der Schraube im unmittelbaren Bereich der Spitze erlaubt ein einfaches Eingreifen der Schraube in den Knochen B. Eine (Vor-)Bohrung wird nicht erstellt, weil die Schraube 2 - wie bereits gesagt - selbstbohrend und auch selbstzentrierend ist. Nach dem Eingreifen der Schraube erfolgt das weitere Einschrauben analog zu Fig. 5, und die dort angestellten Betrachtungen gelten daher auch für dieses zweite Ausführungsbeispiel der Schraube 2.

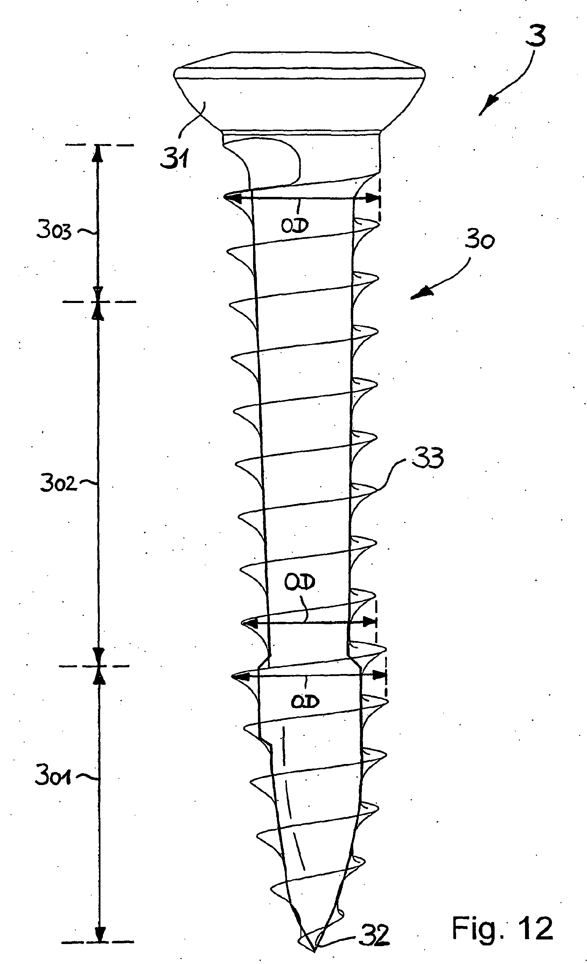

Ein drittes Ausführungsbeispiel der erfindungsgemässen Schraube wird im folgenden anhand von Fig. 12 bis Fig. 16 erläutert. Dieses dritte Ausführungsbeispiel der erfindungsgemässen Schraube 3, erneut eine Knochenschraube, weist in einigen Teilen eine Ähnlichkeit mit dem zweiten Ausführungsbeispiel der erfindungsgemässen Schraube 2 auf, allerdings ist der Vorformbereich bei dem dritten Ausführungsbeispiel der erfindungsgemässen Schraube 3 anders ausgebildet. Grundsätzlich handelt es sich bei der Schraube 3 aber auch um eine selbstbohrende (und natürlich auch selbstschneidende) Schraube. Fig. 12 zeigt eine Ansicht des dritten Ausführungsbeispiels der erfindungsgemässen Schraube 3 - mit übertriebener Darstellung des Kerns des Schraubenschafts. Die Schraube 3 umfasst einen Schaft 30 sowie einen gewindefreien sphärischen Kopf 31, welcher beispielsweise erlaubt, in einem angesenkten Plattenloch einer Knochenplatte (nicht dargestellt) aufgenommen zu werden. Das Schraubenende 32 ist als Spitze ausgebildet.

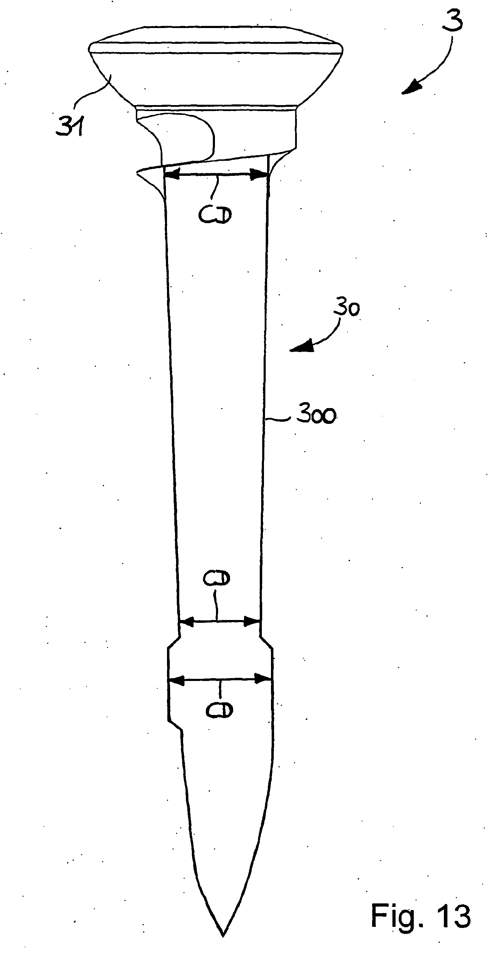

Die Einhüllende über das Gewinde 33 der Schraube, welches hier als durchgängiges Gewinde mit konstanter Steigung ausgebildet ist, kann man anhand von Fig. 12 erahnen, obwohl in Fig. 12 in übertriebener Darstellung der Kern 300 des Schafts 30 dargestellt ist. Der Kern 300 alleine ist noch einmal schematisch in **Fig. 13** dargestellt.

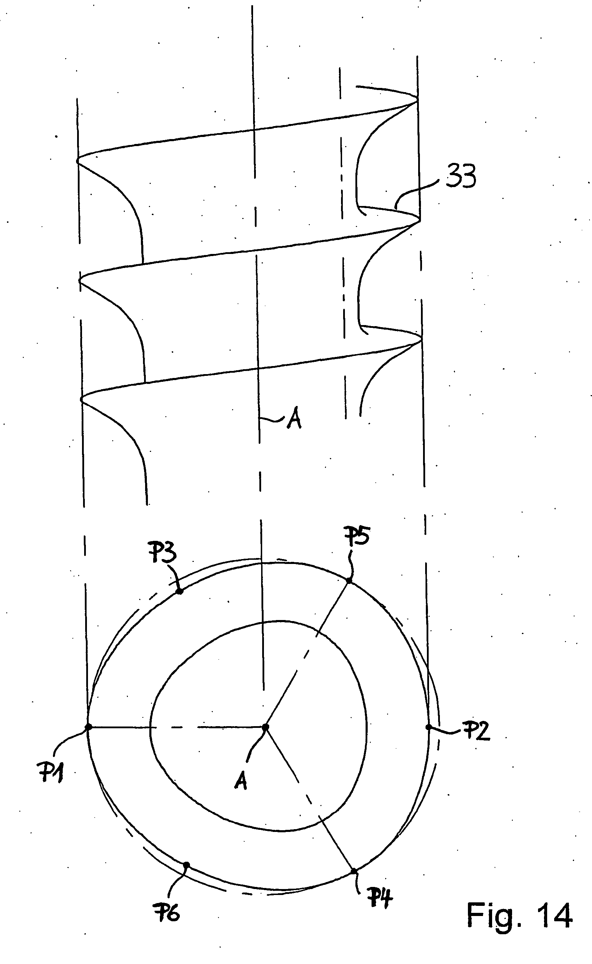

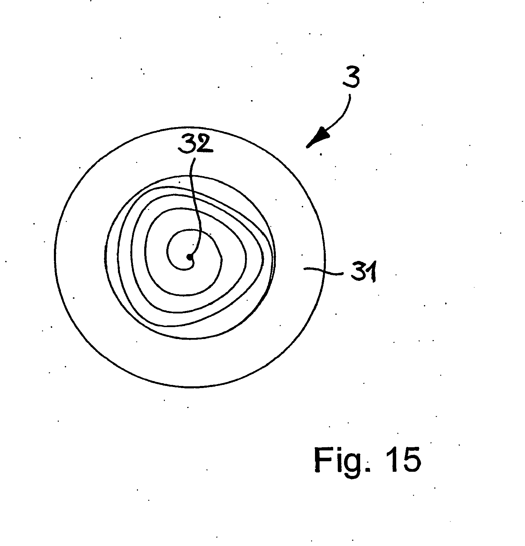

Der Schaft 30 weist erneut drei Bereiche auf: Einen Vorformbereich 301, einen Zwischenbereich 302, sowie einen Verankerungsbereich 303, die man in Fig. 12 erkennen kann. Im Vorformbereich 301 ist nun aber die Schraube 3 anders ausgebildet als bei dem zweiten Ausführungsbeispiel der erfindungsgemässen Schraube 2, nämlich mit einem polygonartigen Querschnitt (siehe auch Fig. 15), hier einem sogenannten "Gleichdick" (was in Fig. 12 durch die gestrichelte Linie im Vorformbereich angedeutet ist). Was unter einem Gleichdick grundsätzlich zu verstehen ist, ist in Fig. 14 besser zu erkennen und wird noch genauer erläutert. In dem unmittelbar an den Zwischenbereich 301 angrenzenden Abschnitt des Vorformbereichs 301 ist der Vorformbereich hingegen nicht als Gleichdick ausgebildet, sondern ähnlich wie beim dem zweiten Ausführungsbeispiel der erfindungsgemässen Schraube, er weist also einen Aussendurchmesser OD auf, der grösser ist als der Aussendurchmesser OD des unmittelbar angrenzenden Abschnitts des Zwischenbereichs 302. Dort ist der Aussendurchmesser OD kleiner als im Vorformbereich 301. Ausgehend von diesem an den Vorformbereich 301 angrenzenden Abschnitt des Zwischenbereichs 302 nimmt der Aussendurchmesser OD über den Zwischenbereich 302 und den Verankerungsbereich 303 hinweg wieder konisch zu, bis er am Beginn des Verankerungsbereichs 303 beinahe gleich gross und am oberen Ende des Verankerungsbereichs 303 gleich gross ist wie in demjenigen Abschnitt des Vorformbereichs 301, der an den Zwischenbereich 302 angrenzt.

Dies gilt analog für den Kerndurchmesser CD des Schafts 30 (siehe Fig. 13). Im unmittelbar an den zwischenbereich angrenzenden Abschnitt des Vorformbereichs ist der Kerndurchmesser CD grösser als in dem unmittelbar daran angrenzenden Abschnitt des Zwischenbereichs, und von dort aus nimmt er über den Zwischenbereich und den Verankerungsbereich hinweg konisch zu.

In Fig. 14 ist nun ein sogenannter "Gleichdick" in der unteren Hälfte dargestellt, und in der oberen Hälfte ein entsprechendes Gewinde, welches entlang seines Umfangs dem Gleichdick folgt. Der Name "Gleichdick" für die Querschnittsform des Schafts in einem Teil des Vorformbereichs resultiert daraus, dass der Durchmesser an zwei gegenüber liegenden Punkten, die sich durch eine Gerade durch die Schraubenachse A verbinden lassen, immer genau gleich gross ist. Somit ist der Schaft bezüglich zweier derart gegenüberliegender Punkte immer gleich dick. Dies erkennt man in Fig. 14 an den gegenüber liegenden Punkten P1, P2 bzw. P3,P4 bzw. P5, P6. Dabei ist zu erkennen, dass die Aussenkontur des Gleichdicks von der Kreisform abweicht, die in Fig. 14 (unten) gestrichelt angedeutet ist. Das zugehörige Gewinde 33 ist in der oberen Hälfte von Fig. 14 dargestellt. Wie man leicht aus der unteren Hälfte von Fig. 14 erkennen kann, gibt es dabei Punkte P1,P4,P5, (bzw. Bereiche) die einen maximalen radialen Abstand von der Achse A der Schraube 3 haben, und Bereiche, die einen geringeren radialen Abstand haben, z.B. die Bereiche, in denen die Punkte P2,P3,P6 liegen. Ein radialer Eingriff des Gewindes mit dem Knochen findet also immer nur in den Punkten P1, P4, P5 (bzw. den Bereichen unmittelbar benachbart zu diesen Punkten) statt. Die eigentliche Gewindeformung findet daher in diesen Punkten bzw. Bereichen statt und gleichzeitig erhöht sich dort die axiale Flächenpressung zwischen Schraube und Knochen. Dadurch lässt sich der Vorformbereich 301 des dritten Ausführungsbeispiels der erfindungsgemässen Schraube 3 noch einmal leichter einschrauben als der Vorformbereich 201 des zweiten Ausführungsbeispiels der erfindungsgemässen Schraube 2.

**Fig. 15** zeigt die Schraube 3 in einer Ansicht von unten, in welcher man gut den Gleichdick von der Spitze 32 der Schraube 3 weg erkennt, bis der Gleichdick dann im oberen Abschnitt des Vorformbereichs an einen Abschnitt mit kreisförmigem Querschnitt anschliesst.

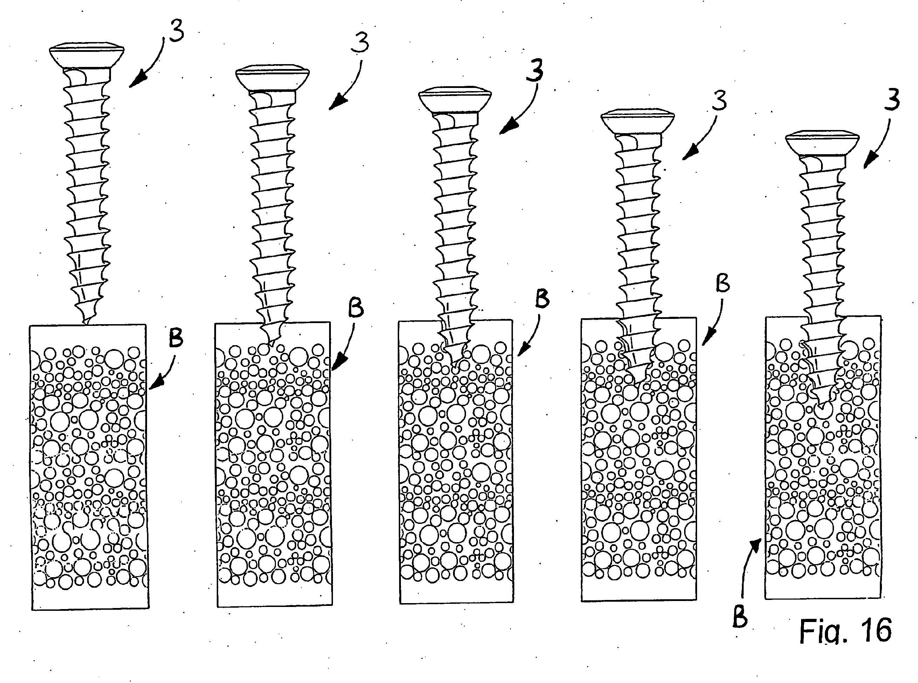

Zum Einschrauben einer solchen Schraube 3, die selbstbohrend ist, wird wie in **Fig. 16** dargestellt vorgegangen. Bezüglich der Beschreibung wird dabei im Wesentlichen auf Fig. 11 verwiesen. Allerdings erkennt man in Fig. 16 gut den Unterschied beim Einschrauben des Vorformbereichs: Die Bereiche, deren radialer Abstand von der Schraubenachse geringer ist (z.B. an den Punkten P2, P3, P6) als der maximale radiale Abstand (z.B. an den Punkten P1, P4, P5) laufen beim Eindrehen in radialer Richtung frei. Dies erkennt man besonders gut in den drei rechten Bildern in Fig. 16.

Zu erwähnen ist noch, dass der Gleichdick nur eines der möglichen Ausführungsbeispiele eines solchen polygonartigen Querschnitts ist - hier kommen auch andere Formen in Betracht (z.B. mehreckige, etc.), wobei die Ecken des jeweiligen Polygons auch gerundet sein können, ähnlich wie bei dem gezeigten Gleichdick.

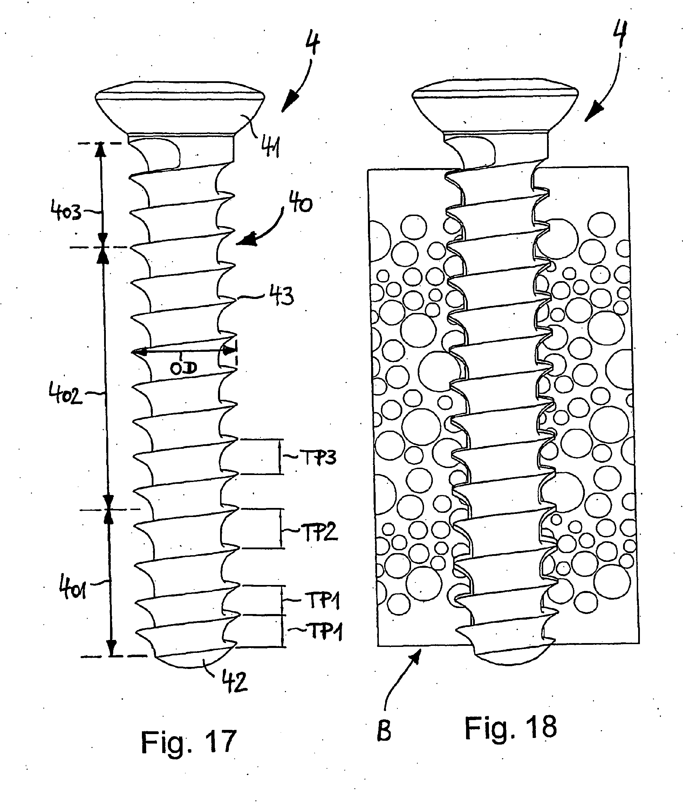

Ein viertes Ausführungsbeispiel der erfindungsgemässen Schraube wird im folgenden anhand von Fig. 17 und Fig. 18 erläutert. In **Fig. 17** ist das vierte Ausführungsbeispiel der erfindungsgemässen Schraube 4, erneut eine Knochenschraube, in einer Ansicht dargestellt. Die Schraube 4 umfasst einen Schaft 40 sowie einen gewindefreien sphärischen Kopf 41, welcher beispielsweise erlaubt, in einem angesenkten Plattenloch einer Knochenplatte (nicht dargestellt) aufgenommen zu werden. Das Schraubenende 42 ist stumpf ausgebildet, es handelt sich somit bei der in Fig. 17 gezeigten Schraube 4 um eine selbstschneidende (nicht selbstbohrende) Schraube.

Die Einhüllende über das Gewinde 43 der Schraube, welches hier als durchgängiges Gewinde ausgebildet ist, verläuft kreiszylindrisch, die Schraube weist also über die gesamte Länge des Schafts 40 einen konstanten Aussendurchmesser OD auf. Ebenso ist der Kerndurchmesser konstant, sodass auch die radiale Gewindehöhe konstant ist.

Der Schaft 40 weist drei Bereiche auf: Einen Vorformbereich 401, einen Zwischenbereich 402, sowie einen Verankerungsbereich 403. Das Gewinde 43 weist im Vorformbereich 401 in einem nahe beim Schraubenende 42 angeordneten Abschnitt eine erste Gewindesteigung TP1 auf, während sie in einem an den Zwischenbereich 402 unmittelbar angrenzenden Abschnitt eine zweite Gewindesteigung TP2 aufweist, die grösser ist als die erste Gewindesteigung TP1. Im Zwischenbereich 402 wie auch im Verankerungsbereich 403 weist das Gewinde 43 dann eine dritte Gewindesteigung TP3 auf, welche grösser als die erste Gewindesteigung TP1, aber kleiner als die zweite Gewindesteigung TP2 ist.

Zum Einschrauben der Schraube 4 in den Knochen B muss zunächst eine (Vor-)Bohrung im Knochen B erstellt werden, wie das anhand von Fig. 5 bereits beschrieben wurde, da die Schraube 4 zwar selbstschneidend, aber eben nicht selbstbohrend ist. Beim Einschrauben des Vorformbereichs 401 wird zunächst durch den Abschnitt mit der Gewindesteigung TP1 ein Knochengewinde mit der Steigung TP1 geformt. Danach wird der Abschnitt mit der Gewindesteigung TP2 eingeschraubt. Die unterschiedlichen Gewindesteigungen TP1 und TP2 sind nun so gewählt, dass nicht ein zusätzliches Knochengewinde mit der Steigung TP2 geschnitten wird, sondern das Knochengewinde mit der Steigung TP1 in axialer Richtung aufgeweitet wird. Das heisst, dass sich die beiden Gewindesteigungen TP1 und TP2 nicht beliebig stark unterscheiden dürfen. In dieses durch den Vorformbereich 401 geformte axial aufgeweitete Knochengewinde gleitet dann das Gewinde 43 des Zwischenbereichs 402 und des Verankerungsbereichs 403 hinein. Da die Gewindesteigung TP3 des Gewindes 43 dort grösser ist als die Gewindesteigung TP1, aber kleiner als die Gewindesteigung TP2, gleiten die Gewindeflanken des Zwischenbereichs 402 und des Verankerungsbereichs 403 quasi ohne axiale Reibung in das von dem Vorformbereich 401 geformte axial aufgeweitete Knochengewinde hinein, sie weisen ein geringes axiales Spiel zum Knochengewinde auf. Dies kann man besonders gut in **Fig. 18** erkennen. In radialer Richtung kann es beim Einschrauben zwar zu Reibung kommen, da der Aussendurchmesser OD der Schraube 4 über die gesamte Länge des Schafts 40 konstant ist. Insgesamt nimmt das zum Einschrauben erforderliche Drehmoment nach dem vollständigen Einschrauben des Vorformbereichs 401 jedoch nur unwesentlich zu, jedenfalls ist es wesentlich geringer als das erforderliche Drehmoment zum Einschrauben konventioneller Schrauben. Dies erleichtert dem Chirurgen das Einschrauben und verhindert ausserdem, dass die Schraube 4 reissen kann (Torsionsbruch). Trotz des geringen axialen Spiels ist die Verankerung der Schraube 4 im Knochen B gut. Der Knochen kann dann ausserdem im weiteren Heilungsverlauf vollständig in das Gewinde einwachsen und das axiale Spiel wird so wieder eliminiert.

Anzumerken ist noch, dass bei transmukosalen oder transgingivalen Anwendungen der erfindunsgemässen Schraube zwischen dem Kopf und dem Verankerungsbereich noch ein vorzugsweise strukturloser (glatter) Bereich der Schraube befinden kann, damit der Kopf der Schraube ausserhalb der Mukosa bzw. Gingiva zu liegen kommt. Der strukturlose (glatte) Bereich verringert dabei das Risiko des Eindringens von Bakterien.

Ausserdem ist natürlich anzumerken, dass von der Schraubenspitze weg im Vorformbereich noch Schneidnuten vorgesehen sein können. Auch der Übergang zwischen dem Vorformbereich und dem Zwischenbereich kann etwas "weicher" (weniger abrupt) ausgebildet sein als bei den dargestellten Ausführungsbeispielen, was insbesondere im Hinblick auf ein späteres Ausdrehen der Schraube von Vorteil sein kann.

Selbstverständlich kann die erfindungsgemässen Schraube auch kanüliert sein, um beispielsweise beim Eindrehen entlang eines zuvor eingebrachten Führungsdrahts geführt zu werden.

In den beschriebenen Ausführungsbeispielen sind eingängige Schrauben gezeigt, aber es ist natürlich auch möglich, dass das Gewinde mehrgängig ausgebildet sein kann, insbesondere dann, wenn die Gewindesteigung konstant ist. Bei mehrgängigen Gewinden kommen insbesondere, aber nicht ausschliesslich, zweigängige oder dreigängige Gewinde in Betracht. Schrauben mit mehrgängigen Gewinden können zu einem noch besseren Halt im Knochen führen, weil sich die Gewindespitzen bzw. Gewindeflanken bei einem mehrgängigen Gewinde (bezogen auf eine bestimmte Azimutebene senkrecht auf die Achse des Schraubenschafts) gleichzeitig an mehreren stellen im Knochen abstützen, was zu einer erhöhten Stabilität der Verankerung im Knochen führt. Beispielsweise liegen die Gewindespitzen bei einer zweigängigen Schraube, bezogen auf eine bestimmte Azimutebene, um einen Azimutwinkel von 180° zueinander versetzt. Bei einer dreigängigen Schraube sind sie jeweils um einen Azimutwinkel von 120° zueinander versetzt.

Weiterhin kann der Kopf der Schraube auch so ausgebildet sein, dass er im Plattenloch einer Knochenplatte verblockt werden kann. Eine besonders bevorzugte Art einer solchen Verblockung, die zwar nicht die einzig mögliche Verblockung ist, aber eine bevorzugte Art der Verblockung, ist z.B. in der WO 2004/086990 beschrieben.

## Patentansprüche

1. Gewinde formende Schraube (1;2;3;4), insbesondere Knochenschraube, mit einem Schraubenende (12;22;32;42), einem Schaft (10;20;30;40) und einem gewindefreien Kopf (11;21;31;41), wobei der Schaft (10;20;30;40) mit einem Gewinde (13;23;33;43) versehen ist und einen Vorformbereich (101;201;301;401), einen Zwischenbereich (102;202;302;402), der dem Vorformbereich (101;201;301;401) nachfolgend angeordnet ist, sowie einen Verankerungsbereich (103;203;303;403) aufweist, der anschliessend an den Zwischenbereich (102;202;302;402) und unterhalb des Kopfs (11;21;31;41) angeordnet ist, wobei der Vorformbereich (101;201;301;401) unmittelbar an das Schraubenende (12;22;32;42) anschliessend angeordnet ist, und wobei beim Einschrauben des Vorformbereichs (101;201;301;401) das zum Einschrauben erforderliche Drehmoment zunimmt, bis der . Vorformbereich (101;201;301;401) vollständig eingeschraubt ist, wobei der Vorformbereich (101;201;301;401), Zwischenbereich (102;202;302;402) und Verankerungsbereich (103;203;303;403) derart ausgebildet sind, dass nach vollständigem Einschrauben des Vorformbereichs (101;201;301;401) entweder keine Zunahme des zum Einschrauben erforderlichen Drehmoments oder nur eine im Verhältnis zur Zunahme beim Einschrauben des Vorformbereichs (101;201;301;401) geringe Zunahme des erforderlichen Drehmoments auftritt, bis die Schraube (1;2;3;4) bis zu ihrem Verankerungsbereich (103;203;303;403) eingeschraubt ist indem der Aussendurchmesser (OD) der Schraube im Vorformbereich (101:201;301) zumindest in einem an den Zwischenbereich (102;202;302) angrenzenden Abschnitt grösser ist als im Zwischenbereich (102;202;302), und dass der Aussendurchmesser (OD) zumindest in einem Teil des Verankerungsbereichs (103;203;303) gleich gross oder im Wesentlichen gleich gross oder grösser ist wie der Aussendurchmesser (OD) des an den Zwischenbereich (102;202;302) angrenzenden Abschnitts des Vorformbereichs (101;201;301) und dass sich über den Vorformbereich (101, 201;301), den Zwischenbereich (102;202;302) sowie den Verankerungsbereich (103;203;303) ein durchgehendes gewinde mit konstanter steigung erstreckt oder indem der Schraubenschaft (40) im Vorformbereich (401) und im Zwischenbereich (402) einen konstanten Aussendurchmesser (OD) aufweist und im Vorformbereich (401) mit einem Gewinde (43) mit verschiedenen Steigungen (TP1, TP2) versehen ist, einer ersten Steigung (TP1) und einer zweiten Steigung (TP2), wobei die zweite Steigung (TP2) grösser ist als die erste Steigung (TP1), und dass der Schraubenschaft (40) im Verankerungsbereich (403) mit einem Gewinde mit einer dritten Steigung (TP3) versehen ist, die grösser ist als die erste Steigung (TP1) und kleiner als die zweite Steigung (TP2) des Gewindes (43) im Vorformbereich (401).

2. Gewinde formende Schraube (1) nach Anspruch 1, bei welcher das Schraubenende (12) stumpf ausgebildet ist und der Aussendurchmesser (OD) im Vorformbereich (101) konstant ist und im Zwischenbereich (102) in einem unmittelbar an den Vorformbereich (101) angrenzenden Abschnitt kleiner ist als der Aussendurchmesser (OD) des Vorformbereichs (101), und bei welcher Schraube der Aussendurchmesser (OD) ausgehend von diesem an den Vorformbereich (101) angrenzenden Abschnitt des Zwischenbereichs (102) über den Zwischenbereich (102) und den Verankerungsbereich (103) hinweg konisch zunimmt, bis er im Verankerungsbereich (103) gleich gross oder im Wesentlichen gleich gross oder grösser ist wie der Aussendurchmesser (OD) des Vorformbereichs (101).

3. Gewinde formende Schraube (2;3) nach Anspruch 1, bei welcher das Schraubenende (22;32) als Spitze ausgebildet ist und der Aussendurchmesser (OD) des Vorformbereichs (202;302) ausgehend von der Spitze bis zu demjenigen Abschnitt, der unmittelbar an den Zwischenbereich (202;302) angrenzt, zunimmt, bei welcher Schraube ferner der Aussendurchmesser (OD) des Zwischenbereichs (202;302) in einem unmittelbar an den Vorformbereich (201;301) angrenzenden Abschnitt kleiner ist als der Aussendurchmesser (OD) des angrenzenden Abschnitts des Vorformbereichs (201;301), und bei welcher Schraube der Aussendurchmesser (OD) ausgehend von diesem an den Vorformbereich (201;301) angrenzenden Abschnitt des Zwischenbereichs (202;302) über den Zwischenbereich (202;302) und den Verankerungsbereich (203;303) hinweg konisch zunimmt, bis er zumindest in einem Teil des Verankerungsbereichs (203;303) gleich gross ist oder im Wesentlichen gleich gross oder grösser wie der Aussendurchmesser (OD) des Vor formbereichs (201;301) in dem an den Zwischenbereich (202;302) angrenzenden Abschnitt.

4. Gewinde formende Schraube (3) nach Anspruch 3, bei welcher der Schraubenschaft (30) im Vorformbereich (301) einen polygonartigen Querschnitt aufweist, der Punkte (P1,P4,P5) bzw. Bereiche aufweist, die einen maximalen radialen Abstand von der Schraubenachse (A) haben und Bereiche (P2,P3,P6), die einen geringeren radialen Abstand von der Schraubenachse (A) haben als diejenigen Punkte (P1,P4,P5) bzw. Bereiche mit maximalem radialen Abstand von der Schraubenachse (A).

5. Gewinde formende Schraube (3) nach Anspruch 4, bei welcher der Schraubenschaft (30) im Vorformbereich als Gleichdick ausgebildet ist.

6. Gewinde formende Schraube (1;2;3) nach einem der Ansprüche 1 bis 5, bei welcher der Kerndurchmesser (CD) des Schraubenschafts (10;20;30) im Zwischenbereich (102;202;302) kleiner ist als in dem an den Zwischenbereich (102;202;302) angrenzenden Abschnitt des Vorformbereichs (101;201;301), und dass in demjenigen Teil des Verankerungsbereichs (103:203:303), der den gleich grossen oder im Wesentlichen gleich grossen oder grösseren Aussendurchmesser (OD) aufweist wie der an den Zwischenbereich (102:202:302) angrenzende Abschnitt des Vorformbereichs (101;201;301), der Kerndurchmesser (CD) des Schraubenschafts (10;20;30) gleich gross oder im Wesentlichen gleich gross oder grösser ist wie der Kerndurchmesser (CD) des an den Zwischenbereich (102;202;302) angrenzenden Abschnitts des Vorformbereichs (101;201;301).

7. Gewinde formende Schraube nach Anspruch 1, bei welcher die Schraube auch im Zwischenbereich (402) mit einem Gewinde versehen ist, dessen Steigung der dritten Steigung (TP3) des Gewindes im Verankerungsbereich (401) entspricht.

8. Gewinde formende Schraube nach einem der vorangehenden Ansprüche, welche aus einem biokompatiblen Material hergestellt ist, beispielsweise aus Titan oder einer Titanlegierung, oder aus einem bioresorbierbaren Material.

## Claims

1. Thread-forming screw (1; 2; 3; 4), in particular a bone screw, having a screw end (12; 22; 32; 42), a shank (10; 20; 30; 40) and a thread-free head (11; 21; 31; 41), wherein the shank (10; 20; 30; 40) is provided with a thread (13; 23; 33; 43) and has a preforming region (101; 201; 301; 401), an intermediate region (102; 202; 302; 402), which is arranged to follow the preforming region (101; 201; 301; 401), and an anchoring region (103; 203; 303; 403), which is arranged so as to adjoin the intermediate region (102; 202; 302; 402) below the head (11; 21; 31; 41), wherein the preforming region (101; 201; 301; 401) is arranged so as to directly adjoin the screw end (12; 22; 32; 42), and wherein, when the preforming region (101; 201; 301, 401) is being screwed in, the torque required for the screwing-in increases until the preforming region (101; 201; 301; 401) has been completely screwed in, wherein the preforming region (101; 201; 301; 401), the intermediate region (102; 202; 302; 402) and the anchoring region (103; 203; 303; 403) are designed in such a way that, after the preforming region (101; 201; 301; 401) has been completely screwed in, there is either no increase in the torque required for the screwing-in or there is only a slight increase in the requisite torque, compared to the increase during the screwing-in of the preforming region (101; 201; 301; 401), until the screw (1; 2; 3; 4) is screwed in up to its anchoring region (103; 203; 303; 403), in that the outside diameter (OD) of the screw in the preforming region (101; 201; 301), at least in a section adjoining the intermediate region (102; 202; 302), is larger than in the intermediate region (102; 202; 302), and in that the outside diameter (OD) at least in part of the anchoring region (103; 203; 303) is the same size as or essentially the same size as or is larger than the outside diameter (OD) of that section of the preforming region (101; 201; 301) which adjoins the intermediate region (102; 202; 302) and in that a continuous thread having a constant pitch extends over the performing region (101; 201; 301), the intermediate region (102; 202; 302) and the anchoring region (103; 203; 303) or in that the screw shank (40) has a constant outside diameter (OD) in the preforming region (401) and in the intermediate region (402) and is provided in the preforming region (401) with a thread (43) having different pitches (TP1, TP2), a first pitch (TP1) and a second pitch (TP2), the second pitch (TP2) being larger than the first pitch (TP1), and in that the screw shank (40) in the anchoring region (403) is provided with a thread having a third pitch (TP3) which is larger than the first pitch (TP1) and smaller than the second pitch (TP2) of the thread (43) in the preforming region (401).

2. Thread-forming screw (1) according to Claim 1, in which the screw end (12) is of blunt design and the outside diameter (OD) is constant in the preforming region (101) and is smaller in the intermediate region (102) in a section directly adjoining the preforming region (101) than the outside diameter (OD) of the preforming region (101), and in which screw the outside diameter (OD), starting from the section of the intermediate region (102) adjoining the preforming region (101), increases conically across the intermediate region (102) and the anchoring region (103) until, in the anchoring region (103), it is the same size as or essentially the same size as or is larger than the outside diameter (OD) of the preforming region (101).

3. Thread-forming screw (2; 3) according to Claim 1, in which the screw end (22; 32) is designed as a point and the outside diameter (OD) of the preforming region (201; 301) increases starting from the point up to the section which directly adjoins the intermediate region (202; 302), in which screw, the outside diameter (OD) of the intermediate region (202; 302) is smaller in a section directly adjoining the preforming region (201; 301) than the outside diameter (OD) of the adjoining section of the preforming region (201; 301), and in which screw the outside diameter (OD), starting from the section of the intermediate region (202; 302) adjoining the preforming region (201; 301), increases conically across the intermediate region (202; 302) and the anchoring region (203; 303) until, at least in one part of the anchoring region (203; 303), it is the same size as or essentially the same size as or is larger than the outside diameter (OD) of the preforming region (201; 301) in the section adjoining the intermediate region (202; 302).

4. Thread-forming screw (3) according to Claim 3, in which the screw shank (30) in the preforming region (301) has a polygon-like cross section which has points (P1, P4, P5) or regions which are at a maximum radial distance from the screw axis (A) and regions (P2, P3, P6) which are at a smaller radial distance from the screw axis (A) than those points (P1, P4, P5) or regions which are at a maximum radial distance from the screw axis (A).

5. Thread-forming screw (3) according to Claim 4, in which the screw shank (30) in the preforming region is designed as a curve of constant width.

6. Thread-forming screw (1; 2; 3) according to one of Claims 1 to 5, in which the core diameter (CD) of the screw shank (10; 20; 30) is smaller in the intermediate region (102; 202; 302) than in the section of the preforming region (101; 201; 301) which adjoins the intermediate region (102; 202; 302), and in which, in the part of the anchoring region (103; 203; 303) whose outside diameter (OD) is the same size as or essentially the same size as or is larger than that section of the preforming region (101; 201; 301) which adjoins the intermediate region (102; 202; 303), the core diameter (CD) of the screw shank (10; 20; 30) is the same size as or essentially the same size as or is larger than the core diameter (CD) of the section of the preforming region (101; 201; 301) which adjoins the intermediate region (102; 202; 302).

7. Thread-forming screw according to Claim 1, in which the screw is also provided in the intermediate region (402) with a thread whose pitch corresponds to the third pitch (TP3) of the thread in the anchoring region (401).

8. Thread-forming screw according to one of the preceding claims, which is produced from a biocompatible material, for example titanium or a titanium alloy, or from a bioresorbable material.

## Revendications

1. Vis apte à former des filets (1; 2; 3; 4), en particulier vis pour os, avec une extrémité de vis (12; 22; 32; 42), une tige (10; 20; 30; 40) et une tête sans filet (11; 21; 31; 41), dans laquelle la tige (10; 20; 30; 40) est pourvue d'un filet (13; 23; 33; 43) et présente une zone de préformage (101; 201; 301; 401), une zone intermédiaire (102; 202; 302; 402) qui suit la zone de préformage (101; 201; 301; 401), ainsi qu'une zone d'ancrage (103; 203; 303; 403), qui est disposée à la suite de la zone intermédiaire (102; 202; 302; 402) et en dessous de la tête (11; 21; 31; 41), dans laquelle la zone de préformage (101; 201; 301; 401) est disposée immédiatement à la suite de l'extrémité de vis (12; 22; 32; 42), et dans laquelle, lors du vissage de la zone de préformage (101; 201; 301; 401), le couple de rotation nécessaire pour le vissage augmente jusqu'à ce que la zone de préformage (101; 201; 301; 401) soit entièrement vissée, dans laquelle la zone de préformage (101; 201; 301; 401), la zone intermédiaire (102; 202; 302; 402) et la zone d'ancrage (103; 203; 303; 403) sont réalisées de telle manière qu'après le vissage complet de la zone de préformage (101; 201; 301; 401), il ne se produise soit aucune augmentation du couple de rotation nécessaire pour le vissage soit seulement une augmentation du couple de rotation nécessaire faible par rapport à l'augmentation lors du vissage de la zone de préformage (101; 201; 301; 401), jusqu'à ce que la vis (1; 2; 3; 4) soit vissée jusque dans sa zone d'ancrage (103; 203; 303; 403), du fait que le diamètre extérieur (OD) de la vis dans la zone de préformage (101; 201; 301) est, au moins dans une partie adjacente à la zone intermédiaire (102; 202; 302), plus grand que dans la zone intermédiaire (102; 202; 302), et du fait que le diamètre extérieur (OD) au moins dans une partie de la zone d'ancrage (103; 203; 303) est égal ou essentiellement égal ou supérieur au diamètre extérieur (OD) de la partie de la zone de préformage (101; 201; 301) adjacente à la zone intermédiaire (102; 202; 302), et du fait qu'un filet continu à pas constant s'étend sur la zone de préformage (101; 201; 301), la zone intermédiaire (102; 202; 302) ainsi que la zone d'ancrage (103; 203; 303), ou du fait que la tige (40) de la vis présente un diamètre extérieur constant dans la zone de préformage (401) et dans la zone intermédiaire (402) et est pourvue dans la zone de préformage (401) d'un filet (43) avec des pas différents (TP1, TP2), un premier pas (TP1) et un deuxième pas (TP2), dans laquelle le deuxième pas (TP2) est plus grand que le premier pas (TP1), et du fait que la tige (40) de la vis est pourvue dans la zone d'ancrage (403) d'un filet avec un troisième pas (TP3), qui est plus grand que le premier pas (TP1) et plus petit que le deuxième pas (TP2) du filet (43) dans la zone de préformage (401).

2. Vis apte à former des filets (1) selon la revendication 1, dans laquelle l'extrémité (12) de la vis est épointée et le diamètre extérieur (OD) dans la zone de préformage (101) est constant, et dans la zone intermédiaire (102), dans une partie immédiatement adjacente à la zone de préformage (101), il est plus petit que le diamètre extérieur (OD) de la zone de préformage (101), et vis dans laquelle le diamètre extérieur (OD) augmente sous forme conique à partir de cette partie de la zone intermédiaire (102) adjacente à la zone de préformage (101) sur l'étendue de la zone intermédiaire (102) et de la zone d'ancrage (103), jusqu'à ce qu'il soit, dans la zone d'ancrage (103), égal ou essentiellement égal ou plus grand que le diamètre extérieur (OD) de la zone de préformage (101).

3. Vis apte à former des filets (2; 3) selon la revendication 1, dans laquelle l'extrémité (22; 32) de la vis est réalisée en forme de pointe et le diamètre extérieur (OD) de la zone de préformage (201; 301) augmente à partir de la pointe jusqu'à la partie qui est immédiatement adjacente à la zone intermédiaire (202; 302), vis dans laquelle en outre le diamètre extérieur (OD) de la zone intermédiaire (202; 302) dans une partie immédiatement adjacente à la zone de préformage (201; 301) est plus petit que le diamètre extérieur (OD) de la partie adjacente de la zone de préformage (201; 301), et vis dans laquelle le diamètre extérieur (OD) augmente en forme de cône à partir de cette partie de la zone intermédiaire (202; 302) adjacente à la zone de préformage (201; 301) sur l'étendue de la zone intermédiaire (202; 302) et de la zone d'ancrage (203; 303), jusqu'à ce qu'il soit, au moins dans une partie de la zone d'ancrage (203; 303), égal ou essentiellement égal ou supérieur au diamètre extérieur (OD) de la zone de préformage (201; 301) dans la partie adjacente à la zone intermédiaire (202; 302).

4. Vis apte à former des filets (3) selon la revendication 3, dans laquelle la tige de vis (30) présente dans la zone de préformage (301) une section transversale polygonale, qui comporte des points (P1, P4, P5) ou des zones, qui présentent une distance radiale maximale par rapport à l'axe (A) de la vis et des zones (P2, P3, P6) qui présentent une distance radiale par rapport à l'axe (A) de la vis plus faible que les points (P1, P4, P5) ou les zones présentant une distance radiale maximale par rapport à l'axe (A) de la vis.

5. Vis apte à former des filets (3) selon la revendication 4, dans laquelle la tige (30) de la vis est réalisée en courbe de largeur constante.

6. Vis apte à former des filets (1; 2; 3) selon l'une quelconque des revendications 1 à 5, dans laquelle le diamètre de noyau (CD) de la tige de vis (10; 20; 30) est plus petit dans la zone intermédiaire (102; 202; 302) que dans la partie de la zone de préformage (101; 201; 301) adjacente à la zone intermédiaire (102; 202; 302), et dans laquelle, dans la partie de la zone d'ancrage (103; 203; 303) qui présente le diamètre extérieur (OD) égal ou essentiellement égal ou supérieur à celui de la zone de préformage (101; 201; 301) adjacente à la zone intermédiaire (102; 202; 302), le diamètre de noyau (CD) de la tige de vis (10; 20; 30) est égal ou essentiellement égal ou supérieur au diamètre de noyau (CD) de la partie de la zone de préformage (101; 201; 301) adjacente à la zone intermédiaire (102; 202; 302).

7. Vis apte à former des filets selon la revendication 1, dans laquelle la vis est pourvue, dans la zone intermédiaire (402), d'un filet dont le pas correspond au troisième pas (TP3) du filet dans la zone d'ancrage (401).

8. Vis apte à former des filets (1) selon l'une quelconque des revendications précédentes, qui est fabriquée en un matériau biocompatible, par exemple en titane ou en un alliage de titane, ou en un matériau bio-résorbable.
